# EUROPEAN PATENT APPLICATION

(11) **EP 0 951 840 A1**
(43) Date of publication of application: **27.10.1999**
(21) Application number: 97122714.5
(22) Date of filing: 23.12.1997
(51) Int. Cl.: A23L 1/23, A23L 1/227

(54) **Process for the preparation of flavouring compositions and use of these compositions in foodstuffs**

(71) Applicant: SOCIETE DES PRODUITS NESTLE S.A., 1800 Vevey (CH)
(72) Inventor: Bel Rhlid, Rachid, 1066 Epalinges (CH); Blank, Imre, 1073 Savigny (CH); Cerny, Christoph, 8400 Winterthur (CH)
(74) Representative: Micheli & Cie

(57) **Abstract**

The invention relates to a process for the preparation of a flavouring composition containing 2-acetyl-2-thiazoline (2-AT) and precursors thereof, as well as additional flavour ingredients such as alkylated-thiazolidines, 2-methyl-3-furanthiol (MFT) and mercaptopentanone, which comprises the bioconversion of a sulfur-containing compound and an organic acid or a derivative thereof in the presence of a yeast. The flavouring composition obtained can be used especially for intensifying the meaty flavour of foodstuffs and petfood.

## Description

The present invention relates to a process for the preparation of flavouring compositions, more particularly the biogeneration of mixtures comprising sulfur-containing compounds and having roasty, meaty-like or sausage-like notes, and which are usable in foodstuffs, especially for intensifying the meaty flavour thereof.

Sulfur-containing compounds are important known flavour constituents in many foods, such as meat. Among these, heterocyclic compounds such as thiazoles and furans, as well as their derivatives play a key role in roasted flavours.

One of these thiazoles with intense roasty flavour is the 2-acetyl-2-thiazoline (2-AT), which was reported first as a volatile constituent of beef broth (J. Agr. Food Chem. vol. 19, No 5, p. 1014-16, 1971) and whose use was thus claimed in DE 1964276 as an agent for improving the aroma of roasted foods.

Various methods are already known to prepare 2-AT or similar thiazole compounds by organic synthesis (Recueil, vol. 91, p. 711-28, 1972) or through the Maillard reaction (J. Agr. Food Chem. vol. 43, No 11, p. 2946-50, 1995), which are however not appropriate for obtaining food-grade products.

Other S-containing compounds are known as constituents of meat flavours and having a meaty and roasty flavour character and a very low sensory threshold value, such as 2-methyl-3-furanthiol (MFT) (see for example U.S. pat. 4,139,649). Also 2-methyl-thiazolidine (MT) has been used in combination with other odorants to enhance meat-like flavours, as reported for example in U.S. pat. 3,881,025.

Therefore, the purpose of this invention is to provide a new and natural route for obtaining food grade complex mixtures containing heterocyclic compounds, especially furan and thiazol derivatives, as well as other sulfur-containing compounds.

The object of the present invention is thus a process for the preparation of a flavouring composition containing 2-acetyl-2-thiazoline (2-AT) and precursors thereof, as well as additional flavour ingredients, such as alkylated thiazolidines, 2-methyl-3-furanthiol (MFT) and mercaptopentanone, which comprises the bioconversion of a sulfur-containing compound and an organic acid or a derivative thereof in the presence of a yeast.

The composition obtained can be used directly as the liquid reaction mixture, or can be submitted to a separation step, so as to recover the supernatant from said reaction mixture.

The process can be carried out under aerobic or anaerobic conditions. The supernatant separated from the reaction mixture, for example by centrifugation, is usable as a flavouring composition either directly in liquid form or in powder from obtained by mild dehydration methods.

The starting sulfur-containing compound can be selected from the group comprising the compounds having the following formula (I) : 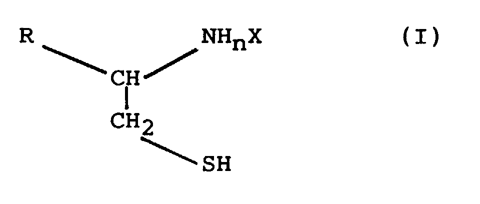 where
- R is H, -COOH, - COOM (M = Na or K), -COONH₄, or -CO-NH-CH₂-COOH,
- X is H, HCl, HBr or -CO-(CH₂)₂-CH(NH₂)-COOH
- and n is 1 or 2,
as well as peptides including said compounds of formula (I).

Among these compounds of formula (I), those preferred are cysteamine and cysteine, or the salts and derivatives thereof, as well as glutathione.

As organic acids, those being of food grade such as the hydroxy- or keto-propionic acids, as well as their derivates, esters and salts thereof, can be used, for example lactic or pyruvic acids, or esters thereof such as ethyl-lactate or ethyl-pyruvate.

The preferred yeast used for the bioconversion is baker's yeast, for example in the form of a powder, an extract or a cream solution, but other kinds of microorganisms can also be used, such as for example Candida versatilis, Debaromyces hansenii, Saccharomyces bayanus, etc. Preferably, the yeast is fresh, up to about 8 days, advantageously up to about 4 days, and kept in the refrigerator.

Regarding the respective quantities of the two starting products, they can be such as the molar ratio between the sulfur-containing compounds and the organic acid is about 1:1 or up to about 1:2. The concentration of these substrates in the reactional medium can be of 1 to 100 mMol, preferably from 10 to 30 mMol.

Generally, the yeast cream solution is used as from 20 to 60 ml per mMol of substrate, but this range can be adjusted according to the yeast and the substrates concerned.

Furthermore, the incubation with yeast can be carried out in presence of a sugar, such as tetroses, pentoses, hexoses, preferably glucose.

Regarding the other reaction conditions, the incubation with the microorganisms can be carried out under aerobic or anaerobic conditions, preferably aerobic during 2 to 72 hr, preferably 4 to 48 hr, and at a pH of 7.0 to 11.0, preferably 8.0 to 10.0. The temperature of the reaction can be of 20 to 50°C, preferably about 30°C, and it can be carried out under medium to high agitation conditions.

The reaction medium can be water or a buffer solution, such as phosphate or carbonate-bicarbonate.

The flavouring composition thus obtained by the process according to the invention surprisingly revealed a flavour described after sensory evaluation as roasty, meaty, sausage-like, dried sausage-like and somewhat amine-like.

The reaction mixture can be used directly as such in liquid form, especially in petfood for aromatising this kind of products.

However, the reaction mixture is advantageously submitted after bioconversion to a separation step, preferably by centrifugation, so as to recover the supernatant from the mycelium. The supernatant can be either maintained as it is in liquid form or dehydrated into a powder under mild conditions, e.g. by spray or freeze drying, this without modifying the aroma thereof.

This composition can be thus advantageously used in foodstuffs in order to intensify the meaty flavour thereof, more particularly in soups, sauces, sausages, meat based dishes and snacks, and other culinary products, as well as in processed meat flavours and petfood.

The flavouring composition can also be combined with other known flavouring agents and/or food additives, possibly in presence of a support such as maltodextrine or cyclodextrine, and thermally processed to increase various types of meaty notes.

The preferred conditions of the optional heat treatment are the following : 80-120°C, 30 to 120 min, and the pH is comprised between 6 and 10, preferably between 6 and 7.

As it will be described thereafter in the Examples, it has been shown by gas chromatography and mass spectrometry analyses that the flavouring composition as obtained by the process according to the present invention, i.e. by biogeneration without heating, is complex and contains among others the compound 2-AT and precursors thereof, as well as other compounds such as MT, MFT, 3-mercapto-2-pentanone, N-acetylcysteamine, 2-ethyl-3,5-dimethyl-pyrazine, etc.

The present invention will now be illustrated by reference to the following Examples.

### Example 1 : Preparation of flavouring Composition A

One liter of commercial yeast cream (from Hefe Schweiz AG) was centrifuged for 15 min at 5000 rpm, and the supernatant was discarded. The mycelium was resuspended in 1 liter of 0.2 M sodium bicarbonate-sodium carbonate buffer at pH 9.8. This yeast cream solution was then used for the bioconversion.

150ml of this yeast cream solution were placed in a 500ml flask equipped with an electrode and a magnetic stirrer (500 rpm). The flask was kept at 35°C using an oil bath and the pH adjusted to 9.8 with 2M sodium hydroxide. The pH was automatically maintained throughout the reaction using a Metrohm pH-stat device. Cysteamine (385mg, 5 mmol) and ethyl-L-lactate (590mg, 5mmol) were then added. 5g and 10g of D-glucose were added after 4 and 24 h of incubation, respectively. After 48h of reaction, the mixture was centrifuged and the supernatant was recovered as Composition A.

### Example 2 : Characterization of Composition A

The main odorant compounds present in Composition A obtained by the process according to the invention have been identified, and the flavour of said composition has been evaluated, more particularly on diethyl-ether extracts. For the extraction, 15ml of the supernatant of Composition A was saturated with NaCl and extracted with purified diethyl-ether, using a liquid-liquid extractor (rotary perforator). The organic phase was dried over anhydrous sodium sulfate and concentrated to about 1ml by micro-distillation.

### A. Gas chromatographic analysis

About twenty odor-active volatile compounds were detected in the aroma extract of Composition A by gaz chromatography-olfactometry (GC-O), using "Carlo Erba" gas chromatographs equipped with automatic cold on-column injector, flame ionization detector and sniffing port, and by gas chromatography-mass spectrometry (CG-MS) using a "Finnigan MAT-8430" apparatus.

Among these about twenty compounds, odorants having an aroma intensity higher than 1 are listed in Table 1, e.g. 2-AT, MT, 2-ethyl-3,5-dimethylpyrazine and N-acetylcystramine, which were identified by GC-O and GC-MS, their sensory and chromatrophic properties being identical to those of the reference compounds. MFT and 3-mercapto-2-pentanone were identified on the basis of retention index on three differently polar capillaries (FFAP, SE-54, OV-1701) and their odour characteristics, which were found to be identical with those of the corresponding reference compounds.

### B. Sensory evaluation

The aroma of Composition A was evaluated by sniffing the headspace of the freshly prepared samples and of diethyl-ether extracts thereof. The assessors (generally 10) were asked to describe the aroma quality and intensity using sniffing strips.

The sensory evaluation made on Composition A resulted in a flavour described as roasty, meaty, amine-like (fishy), dried sausage and sausage-like of high intensity, which indicated that the roasty aroma of 2-AT was at least partly "covered" by other odorants having meaty, sulfury and amine-like notes.

**Table 1**

| Main odorants detected by GC/Olfactometry (CG/O) and GC/MS in the aroma extract of Composition A. | | | | | |
|---|---|---|---|---|---|
| Compound | Identification | | | Aroma quality (GC-O) | Aroma Intensity |
| | RI | MS | Ref. | | |
| Isobutanol | x | x | x | malty | 2 |
| 3-Methyl-1-butanol | x | x | x | metallic, musty, malty | 2 |
| 2-Metyl-3-furanthiol (MFT) (1) | x | - | x | meaty, roasty | 2 |
| 3-Mercapto-2-pentanone (2) | x | - | x | sulfury, catty | 2 |
| 3-Isopropyl-2-methoxypyrazine | x | - | x | roasty | 1 |
| 2-Methylthiazolidine (MT) | x | x | x | amine-like, putrid | 3 |
| 2-Ethyl-3,5-dimethylpyrazine | x | x | x | roasty, earthy | 2 |
| Butanoic acid | x | x | x | sweaty, yeasty | 2 |
| Isovaleric acid | x | x | x | sweaty, rancid, yeasty | 2 |
| 2-Acetyl-2-thiazoline (2-AT) | x | x | x | roasty, popcorn | 2 |
| 2-Phenylethanol | x | x | x | spicy, almond-like | 1 |
| N-Acetylcysteamine | -- | x | -- | burnt, yeasty, musty | 2 |
| RI : Retention Index; MS : Mass Spectrometry; Ref: Reference compound available; Aroma Intensity : 1 (weak), 2 (medium), 3 (high) | | | | | |

| | | | | | |
|---|---|---|---|---|---|
| (1) Ri (SE-54) = 864, Ri (OV-1701) = 932, Ri (FFAP) = 1305 | | | | | |
| (2) Ri (SE-54) = 896, Ri (OV-1701) = 1021, Ri (FFAP) = 1354 | | | | | |

### Example 3 : Drying of Composition A

### A. Freeze-drying of Composition A

1100ml of the liquid preparation Composition A were freeze-dried on plates using a "Lyolab F" apparatus (LSD Secfroid). The liquid was freezed at -40°C and lyophilised in vacuum at 2 mbar. The temperature of the plates was held at 10°C. About 115g of a homogeneous powder was obtained, i.e. Composition AF. For taste-testing, 0,8g of Composition AF and 0,5g table salt were dissolved in 250ml of hot water. The aroma and flavour of the product was described as pleasant sausage-like of high intensity.

### B. Spray-drying of Composition A

35g maltodextrine (Glucidex® 01) were dissolved in 500g of the liquid preparation Composition A. The resulting solution was spray-dried on a "Büchi Mini Spray Dryer" at 115°C (inlet temperature). The powder obtained, as Compositon AS was homogeneous. For taste-testing, 3.4g of Composition AS and 2g table salt were dissolved in 1 lt of hot water and compared with the liquid Composition A (20 g/l). A pronounced and pleasant sausage-like flavour was perceived in both samples.

These trials indicate that Composition A can be dried without changing the aroma character (sausage-like). The resulting powders, Composition AF and AS, are thus suitable for aromatising food products.

### Example 4 : Sausage note (fermented meat note)

A culinary base mass was prepared by adding molten beef fat (6.00 g) to a mixture comprising the following ingredients :

| | |
|---|---|
| Mono Sodium Glutamate | 14.75 g |
| Table Salt | 54.80 g |
| Sodium inosinate | 0.15 g |
| Caramel powder | 0.45 g |
| Curcuma | 0.20 g |
| Rosemary Saromex® | 0.03 g |
| Thyme Saromex® | 0.03 g |
| Laurel Saromex® | 0.03 g |
| Pepper Saromex® | 0.06 g |
| Garlic powder | 0.30 g |
| Onion Saromex® | 1.15 g |
| Corn starch | 7.50 g |
| Yeast extract powder | 3.30 g |
| Maltodextrine | 8.45 g |

After homogenisation, 2.80 g fat powder was added and well mixed, thus yielding a culinary base mass.

A bouillon (reference sample) was prepared by adding 250ml of boiling water to 3.40g of the culinary base mass and 0.10g table salt. The resulting product showed a bouillon flavour, however no pronounced meat character.

To 3.40g of the culinary base mass, 0.50g of the freeze-dried product AF, 0.10g table salt, and 250ml boiling water were added. The resulting bouillon had a very pleasant aroma, which was preferred to the reference sample. The flavour body of the bouillon containing the flavouring ingredient AF was stronger and it had a pronounced fermented meat, sausage-like character, which was absent in the reference sample.

### Example 5 : Meat flavour (beef-type note)

A culinary base mass was prepared by adding molten beef fat (6.00 g) to a mixture comprising the following ingredients :

| | |
|---|---|
| Mono Sodium Glutamate | 10.05 g |
| Table Salt | 38.00 g |
| Sodium inosinate | 0.10 g |
| Caramel powder | 0.40 g |
| Onion Saromex® | 1.00 g |
| Piment Saromex® | 0.15 g |
| Pepper Saromex® | 0.25 g |
| Red Wine powder FIS | 5.00 g |
| Tartaric acid | 0.15 g |
| Corn starch | 5.10 g |
| Yeast extract powder | 2.25 g |
| Maltodextrine | 31.55 g |

The mixture was homogenised and sieved, thus yielding a culinary base mass.

A bouillon (reference sample) was prepared by adding 250 ml of boiling water to 5.00 g of the culinary base mass and 0.10 g table salt. The resulting product showed a good basic bouillon character with a weak beef note.

250 ml boiling water was added to a mixture containing 5.00 g culinary base mass, 0.50 g freeze-dried product AF, and 0.10 g table salt. The resulting bouillon had a very pleasant aroma, which was preferred to the reference sample. The flavour body of the bouillon containing the flavouring ingredient AF was more intense and it had a pronounced beefy, sausage-like character, which was absent in the reference bouillon.

### Example 6 : Meat flavour (chicken-type note)

The following ingredients were mixed :

| | |
|---|---|
| Mono Sodium Glutamate | 10.60 g |
| Sugar | 9.10 g |
| Sodium inosinate | 0.10 g |
| Caramel powder | 0.05 g |
| Onion Saromex® | 0.55 g |
| Rosemary Saromex® | 0.05 g |
| Pepper Saromex® | 0.05 g |
| Ingwer Saromex® S. Jamaica | 0.15 g |
| Cardamon Saromex® | 0.05 g |
| Citric acid | 0.50 g |
| Corn starch | 5.40 g |
| Yeast extract powder | 4.75 g |
| Maltodextrine | 24.25 g |

Chicken fat (5.00 g) was molten together with 0.30 g turmeric, added to 37.80 g table salt and again homogenized. The mixture above was added and again homogenized and sieved. A culinary base mass was prepared by adding 1.30 g fat powder to the mixture.

A bouillon (reference sample) was prepared by adding 250 ml of boiling water to 4.75 g of the culinary base mass and 0.10 g table salt. The resulting product showed a good basic bouillon character with a chicken note.

250ml boiling water was added to a mixture containing 4.75 g culinary base mass, 0.50 g freeze-dried product AF, and 0.10 g table salt. The resulting bouillon containing the flavouring ingredient AF had a very pleasant aroma, still chicken-like, but with a stronger flavour body and a characteristic poultry sausage note. This sample was preferred to the reference bouillon.

### Example 7 : Process flavour (meaty/roasty note)

The following mixture was adjusted with hydrochloric acid (32%) to a pH of 6.5 and then heated in a 500 ml glass reactor with double jacket and stirrer at 100°C for 100 min.

| | |
|---|---|
| Water | 184.5 g |
| Table salt | 123.0 g |
| Soy sauce powder | 61.5 g |
| di-Potassium phosphate | 31.0 g |
| Saccharose | 31.0 g |
| Ribotide® | 31.0 g |
| Palm fat | 18.5 g |
| Cysteine hydrochloride | 11.0 g |
| Dextrose | 11.0 g |
| Xylose | 11.0 g |

After the reaction, 150 g of maltodextrin (Glucidex® 01) was added, homogenized and the mixture was dried in a vacuum oven at 75°C for 3.5 h resulting in Flavour R (reference sample).

A similar meat flavour (Flavour M) was produced using the mixture described above, but replacing water by an equal amount of the liquid preparation Composition A. The reaction and drying conditions were identical.

Each 6.00 g of Flavour R and 6.00 g of Flavour M were dissolved in 500 g boiling water containing 1 g of table salt. The aroma and taste of both products were evaluated. The sample based on Composition A (Flavour M) developed a more pronounced roasty, meaty character compared to the reference sample (Flavour R).

Consequently, as shown in the above Examples, the composition obtained by the process according to the invention can advantageously be used to provide or intensify meaty flavours of foodstuffs, such as soups, sauces, sausages, meat based dishes, snacks, processed flavours or petfood, etc.

## Claims

1. Process for the preparation of a flavouring composition containing 2-acetyl-2-thiazoline (2-AT) and precursors thereof, as well as additional flavour ingredients such as alkylated-thiazolidines, 2-methyl-3-furanthiol (MFT) and mercaptopentanone, which comprises the bioconversion of a sulfur-containing compound and an organic acid or a derivative thereof in the presence of a yeast.

2. Process according to claim 1, wherein the sulfur-containing compound is selected from the group consisting of compounds having the formula (I) : 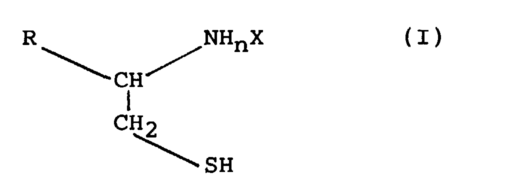 where
- R is H, -COOH, -COOMe (Me = Na or K), -COONH₄, or -CO-NH-CH₂-COOH,
- X is H, HCl, HBr or -CO-(CH₂)₂-CH(NH₂)-COOH
- and n is 1 or 2,
as well as peptides including such compounds of formula (I).

3. Process acording to claims 1 or 2, in which the sulfur-containing compound is cysteamine, cysteine, a salt or derivative thereof, or glutathione.

4. Process according to one of claims 1 to 3, in which the organic acid is a food-grade organic acid, such as hydroxy- or keto-propionic acids, or derivatives, salts or esters thereof.

5. Process according to claim 4, in which said acid is lactic or pyruvic acid, or an ester thereof, for example ethyl-lactate or ethyl-pyruvate.

6. Process according to one of claims 1 to 5, in which the yeast is baker's yeast in the form of a powder, an extract or a cream solution.

7. Process according to one of claims 1 to 6, in which the molar ratio between the sulfur-containing compound and the organic acid is from 1:1 to 1:2.

8. Process according to one of claims 1 to 7, in which the bioconversion is a fermentation, said fermentation being carried out in presence of a sugar, such as tetroses, pentoses or hexoses, preferably glucose.

9. Process according to one of claims 1 to 8, in which the reaction is carried out under aerobic or anaerobic conditions, during 2 to 72 hr, preferably 4 to 48 hr, at a pH of 7.0 to 11.0, preferably 8.0 to 10.0.

10. Process according to one of claim 1 to 9, in which the reaction mixture obtained is submitted to a separation step for recovering the supernatant therefrom.

11. Process according to claim 10, in which the supernatant is dehydrated under mild conditions, for example by freeze or spray drying, and recovered in the form of a powder.

12. Flavouring composition obtainable by the process according to one of claims 1 to 11.

13. Use of the flavouring composition according to claim 12 for intensifying the meaty flavour of foodstuffs.

14. Use of the flavouring composition according to claim 12 in combination with other flavouring agents and/or food additives for intensifying the meaty flavour of foodstuffs and petfood upon heating.

15. Use according to claim 14, in which the heat treatment is carried out at a temperature comprised between 80 and 120°C for 30 to 120 min., and at a pH of 6 to 10, preferably 6 to 7.

16. Use of the flavouring composition obtained by the process according to one of claims 1 to 9, in which the reaction mixture is directly added in liquid form to a food for aromatizing it, especially to petfood.
